Europäisches Patentamt

European Patent Office

Office européen des brevets

(19)

(11) Publication number : **0 352 897 B1**

(12) **EUROPEAN PATENT SPECIFICATION**

(45) Date of publication of patent specification :
01.04.92 Bulletin 92/14

(51) Int. Cl.[5] : **A61K 37/54**

(21) Application number : **89306180.4**

(22) Date of filing : **19.06.89**

(54) TPA-containing medicaments.

(30) Priority : **20.06.88 GB 8814604**

(43) Date of publication of application :
**31.01.90 Bulletin 90/05**

(45) Publication of the grant of the patent :
**01.04.92 Bulletin 92/14**

(84) Designated Contracting States :
**AT BE CH DE ES FR GB GR IT LI LU NL SE**

(56) References cited :
**EP-A- 0 218 112**
**EP-A- 0 252 004**
**FR-A- 2 593 393**

(56) References cited :
CHEMICAL ABSTRACTS, vol. 108, no. 21, 23rd May 1988, page 13, abstract no. 179544d, Columbus, Ohio, US; P. CAMBIER et al.: "Pharmacokinetics and thrombolytic properties of a nonglycosylated mutant of human tissue-type plasminogen activator, lacking the finger and growth factor domains, in dogs with copper coil-induced coronary artery thrombosis", & J. CARDIOVASC. PHARMACOL. 1988, 11(4), 468-72
CHEMICAL ABSTRACTS, vol. 110, no. 11, 13th March 1989, page 51, abstract no. 88316f, Columbus, Ohio, US; J.P. CLOZEL et al.: "Time course of thrombolysis induced by intravenous bolus or infusion of tissue plasminogen activator in a rabbit jugular vein thrombosis model", & CIRCULATION 1989, 79(1), 125-33
Cambier et al 1988 J.Cardvascular Pharm II 468-472 Genetech inc."Activase" product literature

(73) Proprietor : **THE WELLCOME FOUNDATION LIMITED**
**Unicorn House 160 Euston Road**
**London NW1 2BP (GB)**

(72) Inventor : **Cohen, Adam Frederik**
**Julianalaan 9**
**NL-2341 EN Oejstjeest (NL)**

(74) Representative : **Stott, Michael John et al**
**The Wellcome Research Laboratories Group**
**Patents & Agreements Langley Court**
**Beckenham Kent BR3 3BS (GB)**

EP 0 352 897 B1

Jouve, 18, rue Saint-Denis, 75001 PARIS

## Description

The present invention relates to the formulation and administration of tissue plasminogen activator (t-PA).

There exists a dynamic equilibrium between the enzyme system capable of forming blood clots, the coagulation system, and the enzyme system capable of dissolving blood clots, the fibrinolytic system, which maintains an intact patent vascular bed. To limit loss of blood from injury, blood clots are formed in the injured vessel. After natural repair of the injury the superfluous blood clots are dissolved through operation of the fibrinolytic system. Occasionally, blood clots form without traumatic injury and may lodge in major blood vessels resulting in a partial or even total obstruction of blood flow. When this occurs in the heart, lung or brain, the result may be a myocardial infarction, pulmonary embolism or stroke. These conditions combined are the leading cause of morbidity and mortality in the industrialised nations.

Blood clots consist of a fibrous network that is capable of dissolution by the proteolytic plasmin. The enzyme is derived from the inactive proenzyme, plasminogen, a component of blood plasma, by the action of a plasminogen activator. There are two immunologically distinct mammalian plasminogen activators. Intrinsic plasminogen activator, also known as urokinase, is an enzyme produced by the kidney and can be isolated from urine. It can also be prepared from a number of tissue culture sources. Extrinsic plasminogen activator, also known as vascular plasminogen activator and as tissue plasminogen activator (t-PA), can be isolated from many tissue homogenates (notably human uterus), the vascular cell wall and from some cell cultures. In addition to these two kinds of plasminogen activator, there is also a bacterial product, streptokinase, prepared from beta-haemolytic streptococci. A major drawback with both urokinase and streptokinase is that they are active throughout the circulation and not just at the site of a blood clot. They can, for example, destroy other blood proteins, such as fibrinogen, prothrombin, factor V and factor VIII so reducing blood clotting ability and increasing the risk of haemorrhage. In contrast, the biological activity of t-PA is dependent on the presence of fibrin to which it binds and where it is activated. Maximum activity is thus developed only at the site of a blood clot, i.e. in the presence of the fibrin network to be dissolved, and this greatly reduces the risk of haemorrhage.

t-PA is being increasingly used clinically, particularly in the treatment of acute myocardial infarction. Hitherto, t-PA has generally been administered by a small bolus injection followed by a continuous infusion. A typical protocol for treatment with t-PA is as follows:

– Initial bolus injection (about 10% of the lytic dose) over a period of 1-2 minutes
– High level lytic infusion for 1 hour
– Low level maintenance infusion for 2-3 hours.

Bolus injections have also been used in pharmacodynamic studies of the plasma concentration of t-PA (see for example Nilsson et al, Scand. J. Haematol., 33, 49-53 (1984)). However, such studies have indicated that the half life of t-PA in the circulation is less than 5 minutes and it has generally been assumed that most of the dose of t-PA must be administered by continuous infusion in order to maintain adequate concentrations of t-PA in the circulation to achieve thrombolysis. Studies in animals have also suggested that bolus injection has no advantages as a method of administration for t-PA (see for example Gold et al, Circulation, 77(3), 670-677 (1988)).

It has recently been suggested that t-PA and pro-urokinase are complementary in their mode of action and that a combined therapy using the two thrombolytic agents may have advantages. The specific combination was suggested of a bolus injection of t-PA followed by a infusion of pro-urokinase (Gurewich, Journal of the Americal College of Cardiology, Volume 10 No. 5, November 1987, 16B - 21B).

There has not been any suggestion in the case of thrombolytic therapy with t-PA alone that the whole or the dose of t-PA required to achieve thrombolysis should be administered as one or more bolus injections.

There is a continuing need to improve the administration of t-PA particularly in terms of speed and convenience of administration. It has now been found that t-PA can be administered as one or more bolus injections without the need for a following continuous infusion and that this mode of administration shows advantages over modes of administration involving continuous infusion. Despite the fact that the half life of t-PA is about 5 minutes and it is rapidly removed from the circulation, our results suggest that a thrombolytic effect can be produced which outlasts its circulating time.

The present invention provides a method for the manufacture of a medicament for the treatment of a human being with a thrombotic disorder characterised in that t-PA is administered as a bolus injection of at least 12.5MU of t-PA per injection.

The present invention also provides the use of t-PA for the manufacture of a medicament for the treatment of a thrombotic disorder by administering a bolus injection of at least 12.5MU of t-PA per injection.

The use of a bolus injection for the administration of t-PA has a number of advantages over a continuous infusion.

In terms of ease of administration a single injection of low volume via a peripheral vein has considerable advantages over a continuous infusion that requires monitoring. Administration by bolus injection opens up the possibility that the thrombolytic agent t-PA may be administered at the point of first contact with the patient, e.g. in the home, either by paramedical personnel or casualty staff.

Administration by bolus injection also has advantages in terms of clinical efficacy. Thus the time to reperfusion may be minimised in comparison with an infusion, thereby reducing the time that the myocardium remains ischaemic. The optimal dose may well be lower with a bolus injection than that required when administration is by infusion thereby keeping bleeding complications to a minimum. In addition, the shorter duration of administering may well also reduce the dose-related bleeding thought to be associated with prolonged thrombolytic therapy.

When t-PA is administered by continuous infusion it is possible that the clinician may attempt to combine the administration of t-PA with the administration of other therapeutic substances in the same infusion and this may in turn change the bioavailability of the substances thus administered. Bolus injection of t-PA has the advantage that it avoids any possibility that the t-PA may be mixed with other therapeutic substances at the time of administration.

The dose of t-PA when administered by bolus injection may well be lower than the dose for administration by continuous infusion, in which case the cost per patient of therapy with t-PA would be minimised.

Finally administration of t-PA by means of a bolus injection has thrombolytic advantages. The higher blood levels achieved over a short period when the bolus is administered may cause the clot to be saturated with the thrombolytic agent resulting in faster lysis times than can be achieved by the relatively low concentrations administered by continuous infusion. The natural inhibitors of t-PA become rapidly saturated so that peak blood levels of free t-PA available for thrombolysis are reached quickly. In addition the short half life of t-PA means that following a bolus injection high levels of circulating t-PA are not present for prolonged periods. This has the potential to limit intravenous interaction with other therapeutic substances administered for the management of myocardial infarction.

The t-PA of use according to the present invention may be any bioactive protein substantially corresponding to mammalian, and especially human, t-PA and includes forms with and without glycosylation. It may be one- or two-chain t-PA, or a mixture thereof, as described in EP-A-112 122, and, in the case of fully glycosylated human t-PA, has an apparent molecular weight as determined by polyacrylamide gel electrophoresis of about 70,000 and an isoelectric point of between 7.5 and 8.0. Preferably the t-PA has a specific activity of about 0.3 to 0.6MU/mg. As used herein a unit (U) of t-PA activity is the International Unit of activity as defined by the WHO, National Institute for Biological Standards and Control, Holly Hill, Hampstead, London NW3 6RB, United Kingdom and determined by comparison with a standard preparation using a clot-lysis assay.

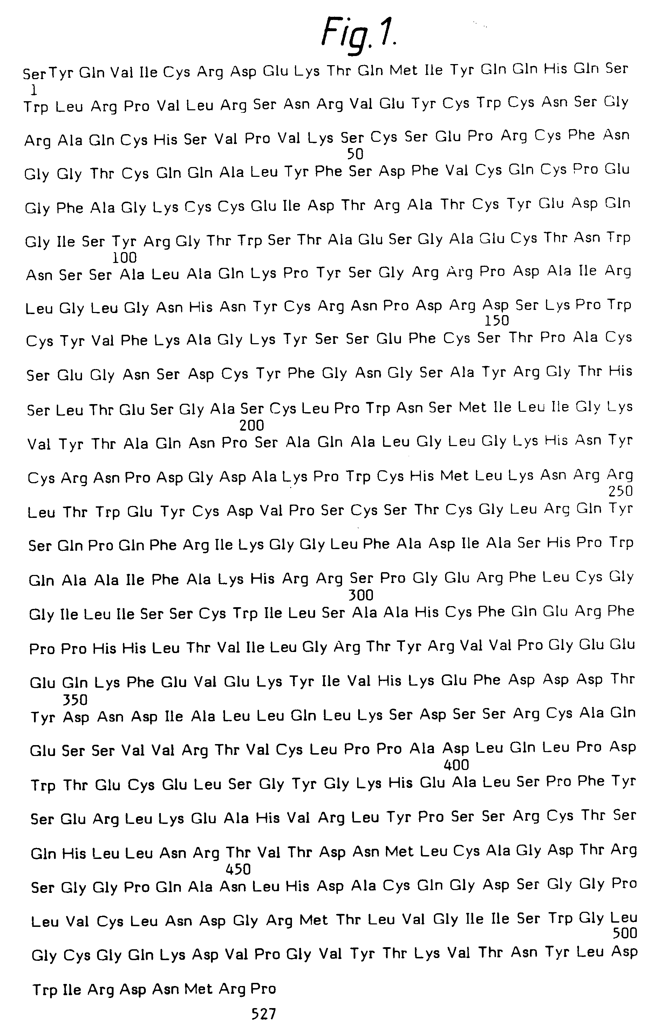

The amino acid sequence of t-PA substantially corresponds to that set forth in Figure 1. The sequence may thus be identical so that in Figure 1 or may contain one or more amino acid deletions, substitutions, insertions, inversions or additions of allelic origin or otherwise, the resulting sequence having at least 90%, homology with the sequence in Figure 1 and retaining essentially the same biological and immunological properties of the protein. In particular, the sequence may be identical to that in Figure 1 or may be the same but with the amino acid in the 245th position from the serine N-terminus being valine instead of methionine, either sequence optionally being without any of the first three amino acids or optionally having an additional polypeptide N-terminal presequence of Gly-Ala-Arg.

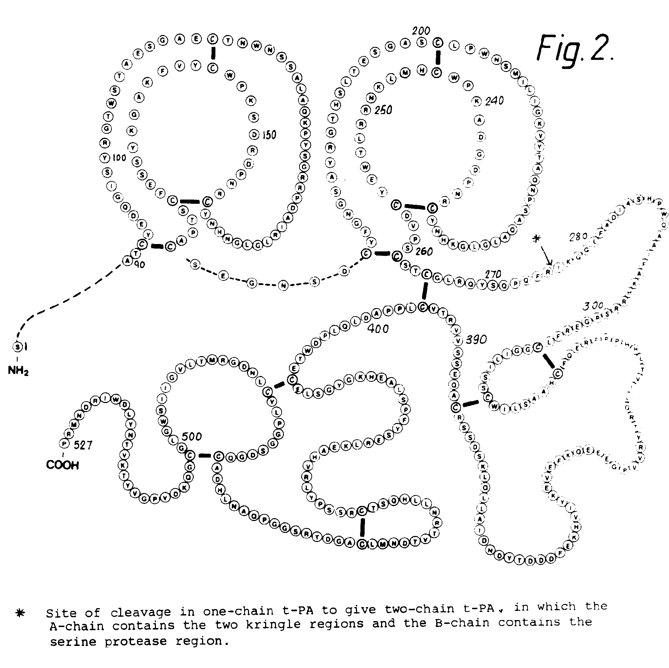

The amino acid sequence set forth in Figure 1 has 35 cysteine residues and thus the potential for forming 17 disulphide bridges. Based on analogy with other proteins whose structure has been determined in more detail, the postulated structure for the sequence (arising from disulphide bond formation) between the amino acid in the 90th position and the proline C-terminus is set forth in Figure 2. The structure of the N-terminal region is less certain although some proposals have been put forward (Progress in Fibrinolysis, 6, 269-293 (1983); and Proc. Natl. Acad. Sci. 81, 5355-5359 (1984)). The most important features of the structure of t-PA are the two kringle regions (between the 93rd and the 173rd amino acids and between the 180th and 261st amino acids), which are responsible for the binding of the protein of fibrin, and the serine protease region, which comprises the major part of the B-chain and which is responsible for the activation of plasminogen. The amino acids of special significance in serine proteases are the catalytic triad, His/Asp/Ser. In t-PA these occur at the 322nd, the 371st and the 463rd positions. The disulphide bridge between the 264th and 395th cysteine amino acid residues is also important in that it holds together the A- and the B- chains in the two chain form of t-PA.

In Figures 1 and 2, the conventional one and three letter codes have been employed for the amino acid residues as follows:

| Asp | D | Aspartic acid | Cys | C | Cysteine |
|-----|---|---------------|-----|---|----------|
| Arg | R | Arginine | Thr | T | Threonine |
| Val | V | Valine | Lys | K | Lysine |
| Ser | S | Serine | Ile | I | Isoleucine |
| Trp | W | Tryptophan | Glu | G | Glutamic acid |
| Leu | L | Leucine | Gln | Q | Glutamine |
| Pro | P | Proline | Tyr | Y | Tyrosine |
| Met | M | Methionine | Gly | G | Glycine |
| Phe | F | Phenylalanine | Asn | N | Asparagine |
| Ala | A | Alanine | His | H | Histidine |

The t-PA may be obtained by any of procedures described or known in the art. For example, it may be obtained from a normal or neoplastic cell line of the kind described in Biochimica et Biophysica Acta, 580, 140-153 (1979); EP-A-766; or EP-A-113 319. It is preferred, however, that t-PA is obtained from a cultured transformed or transfected cell line using recombinant DNA technology as described in, for example, EP-A-93 619; EP-A-117 059; EP-A-117060; EP-A-173 552; EP-A-174 835; EP-A-178 105; EP-A-225 177; EP-A-225 286; WO 86/01538; WO 86/05514; or WO 86/05807. It is particularly preferred that Chinese hamster ovary (CHO) cells are used for the production of t-PA and are derived in the manner as described in Molecular and Cellular Biology, 5(7), 1750-1759 (1985). In this way, the cloned gene is cotransfected with the gene encoding dihydrofolate reductase (dhfr) into dhfr- CHO cells. Transformants expressing dhfr are selected on media lacking nucleosides and are exposed to increasing concentrations of methotrexate. The dhfr and t-PA genes are thus coamplified leading to a stable cell line capable of expressing high levels of t-PA.

The t-PA is preferably purified using any of the procedures described or known in the art, such as the procedure described in Biochimica et Biophysica Acta, 580, 140-153 (1979); J. Biol. Chem., 254(6), 1998-2003 (1979; ibid, 256(13), 7035-7041 (1981); Eur. J. Biochem., 132, 681-686 (1983); EP-A-41 766; EP-A-113 319; or GB-A-2 122 219. Following purification the t-PA is preferably lyophilized most preferably as described in GB-A-2 176 702.

The t-PA will generally be dissolved in a predetermined volume (say up to 10ml per 25MU of t-PA) of sterile water for injection of a suitable alternative carrier such as 5% dextrose. The dose required for bolus injection will then be withdrawn from this volume into a syringe.

In accordance with the invention t-PA is administered as one or more bolus injections of at least 7MU of t-PA per injection. Preferably the t-PA is administered as a single bolus injection of at least 12.5MU of t-PA, for example 25 to 75 MU or 25 to 50 MU of t-PA. The doses given herein are for an average weight human patient of say 70 to 80 kg.

The dose will be given into a peripheral vein for example over a period of 1 to 2 minutes either by direct injection or via an indwelling venous catheter. If given by direct injection into a peripheral vein direct pressure should normally be applied for at least 5 minutes after withdrawing the needle to ensure that haemostasis has occurred.

One particular advantage of administration by bolus injection is that t-PA can be presented in the form of a pre-filled syringe. This simplifies the mode of administration considerably and increases the speed with which thrombolysis can be initiated.

Accordingly the present invention also provides a prefilled syringe containing a unit dose of t-PA in a form suitable for bolus injection. The unit dose of t-PA will generally be at least 12.5 MU, for example 25 to 75 MU or 25 to 50 MU. Such unit doses may also be used for a bolus formulation.

The invention is illustrated by the following Examples.

EXAMPLE 1

A clarified harvest of t-PA obtained from a cultured transformed CHO cell line which was derived using the procedure of Molecular and Cellular Biology, 5(7), 1750-1759 (1985), was purified chromatographically and the t-PA collected as an aqueous solution containing 0.17M sodium citrate and 0.01% (w/v) Tween® 80 at a pH of 5.5. The pH of the solution was adjusted to 3.0 with hydrochloric acid and the resulting solution concentrated by ultrafiltration using an H-10 Cartridge (Amicon Ltd., Upper Hill, Stonehouse, Gloucestershire, England). The concentrated aqueous solution was further purified by applying it to a gel filtration column (Sephadex® G-150; Pharmacia Biotechnology, Uppsala, Sweden) and eluting with 0.85% Saline solution containing 0.01% (w/v) Tween® 80 at a pH of 3.0, and also by using a disposable artificial kidney. The t-PA was precipitated out of solution by increasing the pH to 5.5 with sodium hydroxide and maintaining the suspension at 4°C for 2 hours. The t-PA was recovered by centrifugation at 4000xg for 30 minutes at 4°C. The pellet of t-PA was redissolved in an aqeuous solution of sodium chloride (0.85% w/v) containing 0.01% (w/v) Tween® 80 and adjusted to pH 3.0 with hydrochloric acid. The volume of saline solution used was that required to give a concentration of t-PA between 7MU/ml and 10MU/ml. This solution of t-PA was diluted with further aqueous sodium chloride (0.85% w/v) containing 0.01% (w/v) Tween® 80 and adjusted to pH 3.0 with hydrochloric acid, and also with sufficient of a solution of 10% (w/v) mannitol in the same acid saline solution to give final concentrations of 5MU/ml of t-PA and 25 mg/ml of mannitol. The resulting solution was filter sterilized and dispensed in volumes of 5 ml into glass vials which were frozen at -35°C. A vacuum was applied at 0.05 Torr (0.05 x 1.33 mbar). After about 24 hours, the temperature was gradually increased to 5°C and maintained at this temperature for 16 hours. It was then increased again to 25°C and the vacuum increased to 0.02 Torr (0.02 x 1.33 mbar) for a further 24 hours, after which the vials were sealed under a partial vacuum of 600 Torr (600 x 1.33 mbar) of dry nitrogen.

The vials, containing 25MU of t-PA each, can be used for the preparation of a solution suitable for bolus injection by dissolving the contents of the vial in 5 ml of pyrogen free water for injection Eur. Ph..

## EXAMPLE 2

The administration of t-PA by bolus injection was compared with administration by continuous infusion in an open, randomized pilot study in patients with acute myocardial infarction as defined by characteristic pain and ECG changes. Patients were entered into the study up to six hours after the onset of continuous chest pain. Patients were randomized to receive one or other of the following treatment regimes:

(i) INFUSION - An infusion of 50MU t-PA over the first 1.5 hours followed by an infusion of a reduced rate of 5MU per hour for the next 5 hours even if reperfusion is obtained.

(ii) BOLUS INJECTION - 4 Bolus injections of 12.5MU (each over a period of 2 minutes) at 0, 20, 40 and 60 minutes.

Perfusion grade of the infarct related coronary artery was assessed by coronary arteriography before and at intervals of 15, 35, 55 and 90 minutes after drug administration had commenced. Patency of the artery was assessed over 24 hours by continuous beat-to-beat monitoring of ST segment level and by recatheterization at 24 hours.

The t-PA was produced in accordance with the procedure of Example 1 and was provided as a white lyophilized powder in glass vials, each vial containing 25MU of t-PA. For administration to patients the contents of a vial were dissolved in 5 ml of pyrogen-free water for injection Eur. Ph.. For continuous infusion the solution was further diluted with normal saline or 5% dextrose and used immediately. For bolus injection half of the volume (2.5 ml containing 12.5MU of t-PA) was used at the first point and the other half at the immediately subsequent time point.

Both groups of patients were given an intravenous bolus dose of 5000 IU heparin at the start of cardiac catheterization when the arterial sheath was in place. A continuous intravenous infusion of 1000 IU heparin per hour was started immediately after the 90 minute arteriogram and varied to accomplish an aPTT of 1.5 to 2.0 times normal (laboratory values), and maintained until 4 to 6 hours before removal of arterial and venous sheaths.

Reperfusion was assessed on the scale TIMI grades 0 to 3 (0 = occluded, 3 = complete recanalization). Results on 16 patients (8 in each group) were as follows:

## BOLUS GROUP (n = 8)

7/8 reached TIMI grade 3 at 90 minutes
1/8 failed to reduce recanalize
2/8 late reocclusion at 24 hours
Mean time to reperfusion - 22.4 minutes.

## INFUSION GROUP (n = 8)

5/8 reached TIMI grade 3 at 90 minutes
3/8 failed to recanalize

2/8 recanalized late at 24 hours
Mean time to reperfusion - 39.6 minutes.


## Claims

1. Use of t-PA, having the amino acid sequence set forth in Figure 1 or an amino acid sequence that is at least 90% homologous with that set forth in Figure 1, for the manufacture of a medicament for the treatment of a thrombotic disorder in a human patient by administering a bolus injection of at least 12.5MU of t-PA per injection into a peripheral vein.

2. Use according to claim 1, characterised by the t-PA having the amino acid sequence set forth in Figure 1 or having the same amino acid sequence but with the amino acid in the 245th position from the serine N-terminus being valine instead of methionine.

3. Use according to either of the preceding claims, characterised by the treatment of a myocardial infarction.

4. Use according to any of the preceding claims, characterised by administering from 25 to 75 MU of t-PA per injection.

5. Use according to claim 4, characterised by administering from 25 to 50MU of t-PA per injection.

6. Use according to any one of the preceding claims, characterised by administering a bolus injection as a single bolus injection.

7. Use according to any one of claims 1 to 5, characterised by administering a bolus injection as a serial bolus injection.


## Patentansprüche

1. Verwendung von t-PA mit der in Fig. 1 gezeigten Aminosäuresequenz oder einer Aminosäuresequenz, die mit der in Fig. 1 gezeigten mindestens 90 % homolog ist, zur Herstellung eines Medikaments für die Behandlung einer Thromboseerkrankung in einem menschlichen Patienten durch Verabreichung einer Bolusinjektion von mindestens 12,5 ME pro Injektion in eine periphere Vene.

2. Verwendung nach Anspruch 1, dadurch gekennzeichnet, daß der t-PA die in Fig. 1 gezeigte Aminosäuresequenz oder die gleiche Aminosäuresequenz, wobei aber die Aminosäure in der 245. Stellung vom Serin-N-Terminus Valin anstelle von Methionin ist, aufweist.

3. Verwendung nach einem der vorhergehenden Ansprüche, dadurch gekennzeichnet, daß ein Myokardinfarkt behandelt wird.

4. Verwendung nach einem der vorhergehenden Ansprüche, dadurch gekennzeichnet, daß 25 bis 75 ME t-PA pro Injektion verabreicht werden.

5. Verwendung nach Anspruch 4, dadurch gekennzeichnet, daß 25 bis 50 ME t-PA pro Injektion verabreicht werden.

6. Verwendung nach einem der vorhergehenden Ansprüche, dadurch gekennzeichnet, daß eine Bolusinjektion als eine einzige Bolusinjektion verabreicht wird.

7. Verwendung nach einem der Ansprüche 1 bis 5, dadurch gekennzeichnet, daß eine Bolusinjektion als eine serienweise Bolusinjektion verabreicht wird.


## Revendications

1. Utilisation du t-PA ayant la séquence des acides aminés indiquée à la Fig. 1 ou une séquence d'acides aminés qui a au moins 90% d'homologie avec celle indiquée à la Fig. 1, pour la fabrication d'un médicament pour le traitement d'un trouble thrombotique chez un patient humain, par administration par injection à la seringue d'au moins 12,5 MU de t-PA par injection dans une veine périphérique.

2. Utilisation suivant la revendication 1, caractérisée en ce que le t-PA a la séquence des acides aminés indiquée à la Fig. 1 ou a la même séquence d'acides aminés mais dont l'acide aminé à la 245ème position depuis la sérine N-terminale est la valine au lieu de la méthionine.

3. Utilisation suivant l'une quelconque des revendications précédentes, caractérisée par le traitement de l'infarctus du myocarde.

4. Utilisation suivant l'une quelconque des revendications précédentes, caractérisée par l'administration de 25 à 75 MU de t-PA par injection.

5. Utilisation suivant la revendication 4, caractérisée par l'administration de 25 à 50 MU de t-PA par injection.

6. Utilisation suivant l'une quelconque des revendications précédentes, caractérisée par l'administration par injection à la seringue sous la forme d'une seule injection à la seringue.

7. Utilisation suivant l'une quelconque des revendications 1 à 5, caractérisée par l'administration par injection à la seringue sous la forme d'une série d'injections à la seringue.

# Fig.1.

Ser Tyr Gln Val Ile Cys Arg Asp Glu Lys Thr Gln Met Ile Tyr Gln Gln His Gln Ser
1

Trp Leu Arg Pro Val Leu Arg Ser Asn Arg Val Glu Tyr Cys Trp Cys Asn Ser Gly

Arg Ala Gln Cys His Ser Val Pro Val Lys Ser Cys Ser Glu Pro Arg Cys Phe Asn
50

Gly Gly Thr Cys Gln Gln Ala Leu Tyr Phe Ser Asp Phe Val Cys Gln Cys Pro Glu

Gly Phe Ala Gly Lys Cys Cys Glu Ile Asp Thr Arg Ala Thr Cys Tyr Glu Asp Gln

Gly Ile Ser Tyr Arg Gly Thr Trp Ser Thr Ala Glu Ser Gly Ala Glu Cys Thr Asn Trp
100

Asn Ser Ser Ala Leu Ala Gln Lys Pro Tyr Ser Gly Arg Arg Pro Asp Ala Ile Arg

Leu Gly Leu Gly Asn His Asn Tyr Cys Arg Asn Pro Asp Arg Asp Ser Lys Pro Trp
150

Cys Tyr Val Phe Lys Ala Gly Lys Tyr Ser Ser Glu Phe Cys Ser Thr Pro Ala Cys

Ser Glu Gly Asn Ser Asp Cys Tyr Phe Gly Asn Gly Ser Ala Tyr Arg Gly Thr His

Ser Leu Thr Glu Ser Gly Ala Ser Cys Leu Pro Trp Asn Ser Met Ile Leu Ile Gly Lys
200

Val Tyr Thr Ala Gln Asn Pro Ser Ala Gln Ala Leu Gly Leu Gly Lys His Asn Tyr

Cys Arg Asn Pro Asp Gly Asp Ala Lys Pro Trp Cys His Met Leu Lys Asn Arg Arg
250

Leu Thr Trp Glu Tyr Cys Asp Val Pro Ser Cys Ser Thr Cys Gly Leu Arg Gln Tyr

Ser Gln Pro Gln Phe Arg Ile Lys Gly Gly Leu Phe Ala Asp Ile Ala Ser His Pro Trp

Gln Ala Ala Ile Phe Ala Lys His Arg Arg Ser Pro Gly Glu Arg Phe Leu Cys Gly
300

Gly Ile Leu Ile Ser Ser Cys Trp Ile Leu Ser Ala Ala His Cys Phe Gln Glu Arg Phe

Pro Pro His His Leu Thr Val Ile Leu Gly Arg Thr Tyr Arg Val Val Pro Gly Glu Glu

Glu Gln Lys Phe Glu Val Glu Lys Tyr Ile Val His Lys Glu Phe Asp Asp Asp Thr
350

Tyr Asp Asn Asp Ile Ala Leu Leu Gln Leu Lys Ser Asp Ser Ser Arg Cys Ala Gln

Glu Ser Ser Val Val Arg Thr Val Cys Leu Pro Pro Ala Asp Leu Gln Leu Pro Asp
400

Trp Thr Glu Cys Glu Leu Ser Gly Tyr Gly Lys His Glu Ala Leu Ser Pro Phe Tyr

Ser Glu Arg Leu Lys Glu Ala His Val Arg Leu Tyr Pro Ser Ser Arg Cys Thr Ser

Gln His Leu Leu Asn Arg Thr Val Thr Asp Asn Met Leu Cys Ala Gly Asp Thr Arg
450

Ser Gly Gly Pro Gln Ala Asn Leu His Asp Ala Cys Gln Gly Asp Ser Gly Gly Pro

Leu Val Cys Leu Asn Asp Gly Arg Met Thr Leu Val Gly Ile Ile Ser Trp Gly Leu
500

Gly Cys Gly Gln Lys Asp Val Pro Gly Val Tyr Thr Lys Val Thr Asn Tyr Leu Asp

Trp Ile Arg Asp Asn Met Arg Pro
527

8

Fig. 2.

* Site of cleavage in one-chain t-PA to give two-chain t-PA, in which the A-chain contains the two kringle regions and the B-chain contains the serine protease region.